# EUROPEAN PATENT SPECIFICATION

(11) **EP 1 519 706 B1**
(45) Date of publication and mention of the grant of the patent: **09.04.2008**
(21) Application number: 03735689.6
(22) Date of filing: 26.06.2003
(51) Int. Cl.: A61K 8/34, A61K 8/41, A61Q 5/00

(54) **HAIR TREATMENT COMPOSITIONS**
HAARBEHANDLUNGSZUSAMMENSETZUNGEN
COMPOSITIONS DE TRAITEMENT CAPILLAIRE

(30) Priority: 10.07.2002 EP 02254856
(43) Date of publication of application: 06.04.2005
(73) Proprietor: UNILEVER N.V., 3013 AL Rotterdam (NL); UNILEVER PLC, London EC4P 4BQ (GB)
(72) Inventor: ELLIS, Frances, Ann, Aintree, Liverpool, Merseyside L9 9EQ (GB); SKINNER, Richard, Unilever R & D Port Sunlight, Wirral, Merseyside CH63 3JW (GB)
(74) Representative: Tansley, Sally Elizabeth
(86) International application number: PCT/EP2003/006834
(87) International publication number: WO 2004/006874

(56) References cited:
- EP-A- 0 922 448
- WO-A-00/00164

## Description

### FIELD OF THE INVENTION

The invention relates to hair treatment compositions. More particularly the invention relates to hair treatment compositions comprising certain specific branched hydroxy compounds. The compositions are particularly suitable for application to hair for repair and restoration of damaged hair.

### BACKGROUND AND PRIOR ART

Hair can suffer damage from a number of sources. The hair fibre can be damaged by environmental influences such as exposure to UV and chlorine; chemical influences such as bleaching, perming, overly frequent washing with harsh surfactant-based cleansing shampoo compositions; and mechanical influences such as prolonged use of heated styling appliances.

Damage to the hair typically manifests itself in cuticle and protein loss from the hair fibre, excessive fibre stiffness, hair fibre brittleness and breakage and frayed or split ends.

A wide array of ingredients are claimed to be effective in the treatment and prevention of hair damage, as can be seen from the literature and from marketed products.

Amino acids are commonly used to include protect and repair hair. DE 3118882-A (Noronha RV), describes a tonic for hair which contains a mixture of amino acids. In this mixture, a 1:4:12 histidine:lysine: arginine ratio is said to stimulate keratin formation.

FR 2669224-A (Contier) describes a scalp treatment comprising amino-dicarboxylic acid complexed with a basic amino acid complex as the active ingredient. The complex is said to prevent degradation of the hair root and improve the hydration of the hair keratin by neutralisation of scalp lactic acid. US 4,201,235 (Mare Corp.) describes hair treatment with a "cocktail" of 20 different amino acids and vitamins to enhance softness, lustre and body.

Amine oxides have also been used for the repair and protection of hair use is discussed in co-pending application number PCT/EP01/09275.

The present invention have now found that compositions comprising certain specific branched hydroxy compounds are effective for repairing and preventing the principal symptoms of damaged hair.

### DESCRIPTION OF THE INVENTION

In a first aspect, the present invention provides a hair treatment composition comprising a hair repair compound having the following formula: 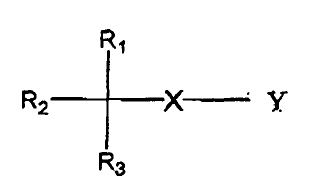 in which R₁, R₂ and R₃ are independently selected from a methyl, ethyl, propyl group or mixtures thereof; X is a substituted or un-substituted alkyl or alkenyl chain and Y is a hydroxy group.

A further aspect of the invention is the use of the above composition for smoothing hair, aligning hair and preventing damage to the hair.

The invention also relates to a method of treating hair by applying the above composition to the hair.

### Hair Repair Compound

The hair repair compound has the general formula: 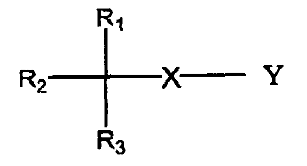 in which R₁, R₂ and R₃ are independently selected from a methyl, ethyl, propyl group or mixtures thereof; X is a substituted or un-substituted alkyl or alkenyl chain and Y is a hydroxy group.

Preferred hair repair compounds comprise at least two hydroxy groups, amino groups or mixtures thereof and have n as the total number of carbon atoms in the molecule such that each OH or NH₂ is positioned with 0.5n or less carbon atoms directly between it and all other OH or NH₂ groups. An example of this would be a molecule having a total of 5 carbon atoms and two hydroxy groups. The hydroxy groups would be 1 or 2 carbon atoms away from each other.

It is preferred if X is substituted with an amino or hydroxy group, particularly a hydroxy group. Especially preferred hair repair compounds are diols.

It is advantageous if the total number of carbon atoms within the hair repair compound is from 5 to 12.

Preferably R₁, R₂ and R₃ of the hair repair compound are all methyl groups.

A particularly preferred hair repair compound is 3, 3-dimethyl-1,2-butandiol.

The total amount of the hair repair compound in hair treatment compositions of the invention is generally from 0.001 to 10 wt%, preferably from 0.01 to 5 wt%, more preferably from 0.05 to 2 wt% and yet more preferably from 0.1 to 1 wt%.

### Product Form

The final product form of hair treatment compositions according to the invention may suitably be, for example, shampoos, conditioners, sprays, mousses, gels, waxes or lotions. Particularly preferred product forms are shampoos, post-wash conditioners (leave-in and rinse-off) and hair treatment products such as hair essences.

The compositions of the invention preferably comprise a cosmetic carrier such as water, alcohol, or surfactant or if a spray a hydrocarbon.

Shampoo compositions preferably comprise one or more cleansing surfactants which are cosmetically acceptable and suitable for topical application to the hair. Further surfactants may be present as emulsifiers.

Suitable cleansing surfactants, are selected from anionic, amphoteric and zwitterionic surfactants, and mixtures thereof. The cleansing surfactant may be the same surfactant as the emulsifier, or may be different.

### Anionic Cleansing Surfactant

Shampoo compositions according to the invention will typically comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alphaolefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl sulpho succinate, ammonium lauryl sulphosuccinate, ammonium lauryl sulphate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n ranges from 1 to 3), ammonium lauryl sulphate and ammonium lauryl ether sulphate(n)EO, (where n ranges from 1 to 3).

The total amount of anionic cleansing surfactant in shampoo compositions of the invention is generally from 5 to 30, preferably from 6 to 20, more preferably from 8 to 16 wt%.

### Co-surfactant

The shampoo composition can optionally include co-surfactants, preferably an amphoteric or zwitterionic surfactant, which can be included in an amount ranging from 0 to about 8, preferably from 1 to 4 wt%.

Examples of amphoteric and zwitterionic surfactants include, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide. Particularly preferred are betaines, in particular cocodimethyl sulphopropyl betaine and preferably lauryl betaine, cocamidopropyl betaine and sodium cocamphopropionate.

Another preferred co-surfactant is a nonionic surfactant, which can be included in an amount ranging from 0 to 8, preferably from 2 to 5 wt%.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain C₅ to C₂₀ alkylor alkenyl group, G is a saccharide group and n is from 1 to 10.

Other sugar-derived nonionic surfactants which can be included in shampoo compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

The shampoo composition can also optionally include one or more cationic co-surfactants included in an amount ranging from 0.01 to 10, more preferably from 0.05 to 5, most preferably from 0.05 to 2 wt%. Useful cationic surfactants are described hereinbelow in relation to conditioner compositions.

The total amount of surfactant (including any co-surfactant, and/or any emulsifier) in shampoo compositions of the invention is generally from 5 to 50, preferably from 5 to 30, more preferably from 10 to 25 wt%.

### Cationic Polymer

A cationic polymer is a preferred ingredient in shampoo compositions of the invention, for enhancing conditioning performance of the shampoo.

The cationic polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.

Suitable cationic nitrogen polymers are described in the CTFA Cosmetic Ingredient Directory, 3rd edition

The cationic conditioning polymers can comprise mixtures of monomer units derived from amine- and/or quaternary ammonium-substituted monomer and/or compatible spacer monomers.

Suitable cationic conditioning polymers include, copolymers of 1-vinyl-2-pyrrolidine and 1-vinyl-3-methylimidazolium salt (CTFA name Polyquaternium-16); copolymers of 1-vinyl-2-pyrrolidine and dimethylaminoethyl methacrylate, (CTFA name Polyquaternium-11); cationic diallyl quaternary ammonium-containing polymers in patricular(CTFA Polyquaternium 6 and Polyquaternium 7, mineral acid salts of amino-alkyl esters of homo-and copolymers of unsaturated carboxylic acids as described in U.S. Patent 4,009,256; cationic polyacrylamides(as described in WO95/22311).

Cationic polysaccharide polymers suitable for use in compositions of the invention include those with an anhydroglucose residual group, such as a starch or cellulose. Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (commercially available from Rhone-Poulenc in their JAGUAR trademark series). Particularly preferred cationic polymers are JAGUAR C13S, JAGUAR C14, JAGUAR C15, JAGUAR C17 and JAGUAR C16 Jaguar CHT and JAGUAR C162.

The cationic conditioning polymer will generally be present in compositions of the invention at levels of from 0.01 to 5, preferably from 0.05 to 1, more preferably from 0.08 to 0.5 wt%.

### Conditioning Surfactant

Conditioner compositions usually comprise one or more conditioning surfactants which are cosmetically acceptable and suitable for topical application to the hair.

Suitable conditioning surfactants are selected from cationic surfactants, used singly or in admixture.

Cationic surfactants useful in compositions of the invention contain amino or quaternary ammonium hydrophilic moieties which are positively charged when dissolved in the aqueous composition of the present invention.

Examples of suitable cationic surfactants are those corresponding to the general formula:

[N(R₁)(R₂)(R₃)(R₄)]⁺ (X)⁻

in which R₁, R₂, R₃, and R₄ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate, nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

The most preferred cationic surfactants for conditioner compositions of the present invention are monoalkyl quaternary ammonium compounds in which the alkyl chain length is C16 to C22.

Examples of suitable cationic surfactants include quaternary ammonium compounds, particularly trimethyl quaternary compounds.

Preferred quaternary ammonium compounds include cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and salts of these where the chloride is replaced by halogen, (e.g. , bromide), acetate, citrate, lactate, glycolate, phosphate, nitrate, sulphate, or alkylsulphate. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC, ex Hoechst Celanese.

Salts of primary, secondary, and tertiary fatty amines are also suitable cationic surfactants. The alkyl groups of such amines preferably have from 12 to 22 carbon atoms, and can be substituted or unsubstituted.

Particularly useful are amido substituted tertiary fatty amines, in particular tertiary amines having one C₁₂ to C₂₂ alkyl or alkenyl chain. Such amines, useful herein, include stearamidopropyIdimethylamine, stearamidopropyidiethylamine, stearamidoethyldiethylamine, stearamidoethyldimethylamine, palmitamidopropyld imethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachid amidopropyldiethylamine, arachidamidoethyldiethylamine, arachidamidoethyldimethylamine, diethylaminoethylstearamide. Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

These amines are typically used in combination with an acid to provide the cationic species. The preferred acid useful herein includes L- glutamic acid, lactic acid, hydrochloric acid, malic acid, succinic acid, acetic acid, fumaric acid, tartaric acid, citric acid, L-glutamic hydrochloride, and mixtures thereof; more preferably L-glutamic acid, lactic acid, citric acid. Cationic amine surfactants included among those useful in the present invention are disclosed in U.S. Patent 4,275,055 to Nachtigal, et al., issued June 23, 1981.

The molar ratio of protonatable amines to H⁺ from the acid is preferably from about 1:0.3 to 1:1.2, and more preferably from about 1:0.5 to about 1:1.1.

In the conditioners of the invention, the level of cationic surfactant is preferably from 0.01 to 10, more preferably 0.05 to 5, most preferably 0.1 to 2 wt% of the total composition.

The cationic surfactants detailed in this section are also suitable for use in the aspect of the invention wherein a cationic surfactant is intimately mixed with the thermotropic mesogenic material and with oily conditioning material prior to the incorporation of the conditioning material into the final hair conditioning composition

### Fatty Materials

Conditioner compositions of the invention preferably additionally comprise fatty materials. The combined use of fatty materials and cationic surfactants in conditioning compositions is believed to be especially advantageous, because this leads to the formation of a structured phase, in which the cationic surfactant is dispersed.

By "fatty material" is meant a fatty alcohol, an alkoxylated fatty alcohol, a fatty acid or a mixture thereof.

Preferably, the alkyl chain of the fatty material is fully saturated.

Representative fatty materials comprise from 8 to 22 carbon atoms, more preferably 16 to 22. Examples of suitable fatty alcohols include cetyl alcohol, stearyl alcohol and mixtures thereof. The use of these materials is also advantageous in that they contribute to the overall conditioning properties of compositions of the invention.

Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty alcohol material in conditioners of the invention is suitably from 0.01 to 15, preferably from 0.1 to 10, and more preferably from 0.1 to 5 wt%. The weight ratio of cationic surfactant to fatty alcohol is suitably from 10:1 to 1:10, preferably from 4:1 to 1:8, optimally from 1:1 to 1:7, for example 1:3.

### OPTIONAL INGREDIENTS

### Suspending Agents

In a preferred embodiment, the hair treatment composition, especially if it is a shampoo composition, further comprises from 0.1 to 5 wt% of a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 941 and Carbopol 980. An example of a suitable copolymer of a carboxylic acid containing a monomer and acrylic acid esters is Carbopol 1342. All Carbopol (trade mark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

### Conditioning Agents

### Silicone Conditioning Agents

The compositions of the invention can contain, emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the shampoo compositions of the invention will typically have an average silicone droplet size in the composition of less than 30, preferably less than 20, more preferably less than 10 µm, ideally from 0.01 to 1 µm. Silicone emulsions having an average silicone droplet size of ≤ 0.15 µm are generally termed microemulsions.

Examples of suitable pre-formed emulsions include emulsions DC2-1766, DC2-1784, DC-1785 DC-1786 and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone", Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones).
Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions Doc2-7224, DC2-8467, DC2-8177 and DC2-8154 (all ex Dow Corning).

With some shampoos it is particularly preferred to use a combination of amino and non amino functional silicones

The total amount of silicone is preferably from 0.01 to 10 %wt of the total composition more preferably from 0.3 to 5, most preferably 0.5 to 3 wt% is a suitable level.

### (ii) Non-silicone Oily Conditioning Components

Compositions according to the present invention may also comprise a dispersed, non-volatile, water-insoluble oily conditioning agent.

By "insoluble" is meant that the material is not soluble in water (distilled or equivalent) at a concentration of 0.1% (w/w), at 25°C.

Suitable oily or fatty materials are selected from hydrocarbon oils, fatty esters and mixtures thereof. Straight chain hydrocarbon oils will preferably contain from about 12 to about 30 carbon atoms. Also suitable are polymeric hydrocarbons of alkenyl monomers, such as C₂-C₆ alkenyl monomers.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and triesters of glycerol and long chain carboxylic acids such as C₁-C₂₂ carboxylic acids. Preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3 wt%.

In hair treatment compositions containing a conditioning agent, it is preferred that a cationic polymer also be present.

### Adjuvants

The compositions of the present invention may also contain adjuvants suitable for hair care. Generally such ingredients are included individually at a level of up to 2, preferably up to 1 wt% of the total composition.

Suitable hair care adjuvants, include amino acids, sugars and ceramides.

The invention will now be further illustrated by the following, non-limiting Examples.

Examples of the invention are illustrated by a number, Comparative Examples are illustrated by a letter. All percentages quoted are by weight based on total weight unless otherwise stated.

### EXAMPLES

### Example 1

The following base shampoo compositions were prepared:

| | **Examples** | | | |
|---|---|---|---|---|
| | **A** | **B** | **1** | **2** |
| Sodium lauryl ether sulphate(2EO) | 12 | 12 | 12 | 12 |
| Cocoamidopropyl betaine | 2 | 2 | 2 | 2 |
| Water | to 100 | to 100 | To 100 | to 100 |

To these base solutions 0.03M of the following compounds were added:
Compound B is 2-methyl-2,4 pentanediol
Compound 1 is 3,3-dimethylbutan-1-ol
Compound 2 is 3,3-dimethyl-1,2-butanediol.

The compositions were tested in a cuticle abrasion test. Root end hair (as received from the supplier) was pre-cleaned by washing for 1 minute in Composition I followed by copious rinsing in double deionised water. The hair was left to dry naturally over 24 hrs.

For each test, 4 g of hair was washed in 0.4 g of the test composition for 30 seconds, rinsed for 30 seconds, and the washing/rinsing cycle repeated. The hair was left to dry naturally overnight.

The hair was cut into 1 cm lengths, placed in a blender bucket and 200 ml of double de-ionised water was added. The blender was placed on full power for 1 minute. The liquor was decanted off and transferred to a 50 ml centrifuge tube and centrifuged for 10 minutes at 4,300 RPM. The resulting clear liquor was decanted off leaving the cuticle sediment at the bottom of the tube.

The cuticle was transferred from the tube into a beaker with 3 x 5 ml washes and passed through a series of sieves (425 and 53 microns). The sieves were washed with a further 2 x 10 ml of water to ensure complete recovery of the cuticle. The sample was made up to 50 ml in a centrifuge tube, centrifuged for 15 minutes and the liquor decanted off. The cuticle was transferred with double deionised water (2 x 10 ml washes) to a beaker and freeze-dried.

The freeze-dried cuticle was weighed back to determine percentage recovery.

The results were as follows:

| | Example | | | |
|---|---|---|---|---|
| | A | B | 1 | 2 |
| % recovery | 0.50 | 0.50 | 0.31 | 0.27 |

In conclusion, Compositions 1 and 2 perform better than comparative Examples A and B.

### Example 3

The following shampoo composition was prepared:

| | Composition 3 (wt%) |
|---|---|
| Sodium lauryl ether sulphate (2EO) | 12.0 |
| Cocoamidopropyl betaine | 2.1 |
| Dimethiconol emulsion | 1.5 |
| Jaguar C13S | 0.3 |
| Compound 2 | 0.2 |
| Other ingredients | 3.2 |
| Aqua | to 100 |

The dimethiconol emuslsion was DC2-1784 Dow Corning

Jaguar C13s is Guar hydroxypropyl trimonium chloride.

### Example 4

The following conditioner was prepared:

| Ingredient | Trade name | Supplier | %w/w |
|---|---|---|---|
| Deionised water | | | q.s. to 100 |
| Compound 2, | | | 0.5 |
| Behenyl trimethyl ammonium chloride | Genamin KDM-P | Clairant | 1 |
| Behenyl alcohol | | | 5 |
| Silicone emulsion | DC2-1784 | Dow Corning | 2 |
| Disodium EDTA | | | 0.1 |
| Methyl paraben | Nipagin | Nipa Lab | 0.2 |
| Perfume | | | 0.5 |

Compound 2 as for Example 3.

## Claims

1. A hair treatment composition comprising 0.01 to 5 wt. % of a molecule having the following formula: 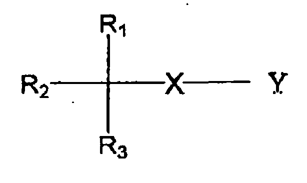 in which R₁, R₂ and R₃ are independently selected from a methyl, ethyl, propyl group or mixtures thereof; X is a substituted or un-substituted alkyl or alkenyl chain and Y is a hydroxy group.

2. A hair treatment composition according to claim 1 in which X is substituted with an amino or hydroxy group.

3. A hair treatment composition according to any preceding claim in which the total number of carbon atoms in the molecule is from 5 to 12.

4. A hair treatment composition according to any preceding claim in which the molecule comprises at least two hydroxy groups, amino groups or mixtures thereof and where n is the total number of carbon atoms in the molecule such that each OH or NH₂ is positioned with 0.5n or less carbon atoms directly between it and all other OH or NH₂ groups.

5. A hair treatment composition according to any preceding claim in which R₁, R₂ and R₃ are all methyl groups.

6. A hair treatment composition according to any preceding claim in which the molecule is a diol.

7. A hair treatment composition according to any preceding claim in which the molecule is 3,3-dimethyl-1,2-butandiol.

8. A hair treatment composition according to any preceding claim further comprising a surfactant.

9. A hair treatment composition according to any preceding claim which further comprises a betaine.

10. A hair treatment composition according to any preceding claim which comprises an aqueous base.

11. Use of a composition according to any preceding claim for smoothing hair.

12. Use of a composition according to any one of claims 1 to 10 for aligning hair.

13. Use of a composition according to any one of claims 1 to 10 for preventing damage to the hair.

14. A method of treating hair by applying a composition according to any one of claims 1 to 10 to the hair.

## Patentansprüche

1. Haarbehandlungszusammensetzung, umfassend 0,01 bis 5 Gewichtsprozent von einem Molekül der nachstehenden Formel: 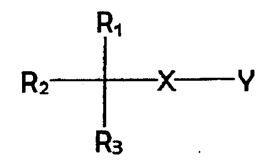 worin R₁, R₂ und R₃ unabhängig aus einer Methyl-, Ethyl-, Propylgruppe oder Gemischen davon ausgewählt sind; X eine substituierte oder unsubstituierte Alkyl- oder Alkenylkette darstellt und Y eine Hydroxygruppe darstellt.

2. Haarbehandlungszusammensetzung nach Anspruch 1, worin X mit einer Amino- oder Hydroxygruppe substituiert ist.

3. Haarbehandlungszusammensetzung nach einem vorangehenden Anspruch, worin die Gesamtanzahl an Kohlenstoffatomen in dem Molekül 5 bis 12 ist.

4. Haarbehandlungszusammensetzung nach einem vorangehenden Anspruch, worin das Molekül mindestens zwei Hydroxygruppen, Aminogruppen oder Gemische davon umfasst, und worin n die Gesamtanzahl an Kohlenstoffatomen in dem Molekül ist, sodass jedes OH oder NH₂ mit 0,5 n oder weniger Kohlenstoffatomen direkt zwischen ihm und allen anderen Gruppen OH oder NH₂ angeordnet ist.

5. Haarbehandlungszusammensetzung nach einem vorangehenden Anspruch, worin R₁, R₂ und R₃ alle Methylgruppen darstellen.

6. Haarbehandlungszusammensetzung nach einem vorangehenden Anspruch, worin das Molekül ein Diol ist.

7. Haarbehandlungszusammensetzung nach einem vorangehenden Anspruch, worin das Molekül 3,3-Dimethyl-1,2-butandiol ist.

8. Haarbehandlungszusammensetzung nach einem vorangehenden Anspruch, weiterhin umfassend ein Tensid.

9. Haarbehandlungszusammensetzung nach einem vorangehenden Anspruch, weiterhin umfassend ein Betain.

10. Haarbehandlungszusammensetzung nach einem vorangehenden Anspruch, die eine wässrige Base umfasst.

11. Verwendung einer Zusammensetzung nach einem vorangehenden Anspruch zum Glätten von Haar.

12. Verwendung einer Zusammensetzung nach einem der Ansprüche 1 bis 10 zum Ausrichten von Haar.

13. Verwendung einer Zusammensetzung nach einem der Ansprüche 1 bis 10 zum Verhindern von Schädigung des Haars.

14. Verfahren zum Behandeln von Haar durch Auftragen einer Zusammensetzung nach einem der Ansprüche 1 bis 10 auf das Haar.

## Revendications

1. Composition pour le traitement des cheveux comprenant de 0,01 à 5 % en poids d'une molécule ayant la formule suivants : 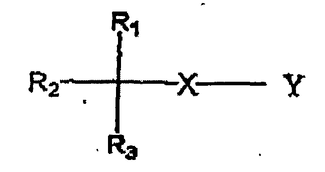 dans laquelle R₁, R₂ et R₃ sont indépendamment choisis parmi un groupe méthyle, éthyle, propyle ou des mélanges de ceux-ci ; X est une chaîne alkyle ou alcényle substituée ou non substituée et Y est un groupe hydroxyle.

2. Composition pour le traitement des cheveux selon la revendication 1, dans laquelle X est substitué par un groupe amino ou hydroxyle.

3. Composition pour le traitement des cheveux selon l'une quelconque des revendications précédentes dans laquelle le nombre total d'atomes de carbone dans la molécule va de 5 à 12.

4. Composition pour le traitement des cheveux selon l'une quelconque des revendications précédentes dans laquelle la molécule comprend au moins deux groupes hydroxyles, groupes aminos ou mélanges de ceux-ci et où n est le nombre total d'atomes de carbone dans la molécule de telle sorte que chaque groupe OH ou NH₂ est positionné avec 0,5n ou moins atomes de carbone directement entre lui-même et tout autre groupe OH ou N2.

5. Composition pour le traitement des cheveux selon l'une quelconque des revendications précédentes, dans laquelle R₁, R₂ et R₃ sont tous des groupes méthyles.

6. Composition pour le traitement des cheveux selon l'une quelconque des revendications précédentes, dans laquelle la molécule est un diol.

7. Composition pour le traitement des cheveux selon l'une quelconque des revendications précédentes, dans laquelle la molécule est le 3,3-diméthylbutane-1,2-diol.

8. Composition pour le traitement des cheveux selon l'une quelconque des revendications précédentes, comprenant en outre un agent tensioactif.

9. Composition pour le traitement des cheveux selon l'une quelconque des revendications précédentes, comprenant en outre une bétaïne.

10. Compositions pour le traitement des cheveux selon l'une quelconque des revendications précédentes, comprenant une base aqueuse.

11. Utilisation d'une composition selon l'une quelconque des revendications précédentes pour lisser les cheveux.

12. Utilisation d'une composition selon l'une quelconque des revendications 1 à 10 pour aligner les cheveux.

13. Utilisation d'une composition selon l'une quelconque des revendications 1 à 10 pour éviter l'endommagement des cheveux.

14. Procédé de traitement des cheveux par application d'une composition selon l'une quelconque des revendications 1 à 10 aux cheveux.
